# EUROPEAN PATENT APPLICATION

(11) **EP 4 316 516 A1**
(43) Date of publication of application: **07.02.2024**
(21) Application number: 22780975.3
(22) Date of filing: 29.03.2022
(51) Int. Cl.: A61K 39/145, A61P 31/16, A61K 9/08, A61K 9/14, C12N 7/04

(54) **INFLUENZA VACCINE FOR TRANSNASAL ADMINISTRATION**

(30) Priority: 30.03.2021 JP 2021057382
(71) Applicant: Denka Company Limited, Tokyo 103-8338 (JP); National University Corporation Kumamoto University, Kumamoto-shi, Kumamoto, 860-0862 (JP)
(72) Inventor: MITSUMATA, Ryotaro, Gosen-shi, Niigata 959-1695 (JP); MISUMI, Shogo, Kumamoto-shi, Kumamoto 860-0862 (JP); NAKATA, Nagisa, Gosen-shi, Niigata 959-1695 (JP); KISHIMOTO, Naoki, Kumamoto-shi, Kumamoto 860-0862 (JP)
(74) Representative: Wächtershäuser & Hartz Patentanwaltspartnerschaft mbB
(86) International application number: PCT/JP2022/015639
(87) International publication number: WO 2022/210764

(57) **Abstract**

Provided is an influenza vaccine for intranasal administration that is efficiently taken up through a nasal mucosa. An influenza vaccine composition to be intranasally administered, comprising an influenza antigen to which TGDK is linked via a chemical bond.

## Description

### Technical Field

The present invention relates to an influenza vaccine for intranasal administration that is efficiently taken up through nasal mucosa.

### Background Art

An influenza virus circulating in winter is an acute infection that infects an upper respiratory tract and presents with sudden fever, cough, and sore throat. As a preventive method against the influenza virus, vaccines can be exemplified; however, most of the vaccines approved worldwide are injectable vaccines, and a systemic immune response represented by an antibody response in blood can be promoted, but mucosal immunity in an infected area by the virus cannot be induced.

In Europe and the United States, a live attenuated vaccine for intranasal administration that has a higher protective effect of infection than an approved injectable vaccine has been approved for children who account for majority of infected people and have a high risk of encephalopathy, and it is known that intranasal vaccines exhibit an excellent effect on upper respiratory tract infections. However, the live attenuated vaccine (Non Patent Literature 1) approved for pediatric use in Europe and the United States has a high efficacy as well as strong side reactions, and therefore the live attenuated vaccine is applied to children of 2 years old or more, but cannot be administered to infants under 2 years old that have a highest risk of exacerbation.

In Japan, in order to solve these problems of side reactions, research and development of a preparation for intranasal administration of a whole inactivated virus vaccine have been advanced, and it has been reported that not only an antibody response in blood but also an IgA in nasal mucosa can be induced by the intranasal administration of the whole inactivated virus vaccine in clinical practice (Non Patent Literature 2). However, a dose of the whole inactivated virus vaccine administered clinically is 45 µg HA/Strain/shot that is 3 times a dose of the approved vaccine, and sufficient immunity induction cannot be achieved in the intranasal administration of the whole inactivated virus vaccine unless a high-titer vaccine is used. This is because mucosal epithelial cells constantly exposed to an external environment have a barrier function.

One way to efficiently take the vaccines into a body beyond a barrier of mucosal epithelium is delivery of the vaccines via M cells. M cells are cells that sample foreign antigens, efficiently deliver the foreign antigens to immunocompetent cells located immediately below, and contribute to modulation of the mucosal immunity. Several substances that target and specifically bind to M cells are known, and examples of the substances include invasin that Yersinia bacteria has and σ1 protein that is a surface antigen of a rotavirus, but all of the substances are foreign proteins, and for these proteinaceous target molecules, there is concern about the immunity induction to the target molecules.

The present inventors have found, as shown in Patent Literatures 1 and 2 and Non Patent Literature 3, tetragalloyl-D-lysine dendrimer (TGDK), which is a gallic acid derivative, as a non-proteinaceous M cell-targeting molecule, and proved that the targeting molecule targets M cells in the intestinal tract. However, it has not been known that TGDK exhibit similar functions in the intranasal administration as those in the intestinal tract, and it also has not been known that combination of TGDK and the influenza vaccine is effective.

### Citation List

### Patent Literature

Patent Literature 1: WO 2007/052641 A
Patent Literature 2: WO 2013/024859 A

### Non Patent Literature

Non Patent Literature 1: Mossad SB. Demystifying FluMist, a new intranasal, live influenza vaccine. Cleve Clin J Med. 2003 Sep;70(9):801-6.
Non Patent Literature 2: Ainai A, Tamura S, Suzuki T, et al. Intranasal vaccination with an inactivated whole influenza virus vaccine induces strong antibody responses in serum and nasal mucus of healthy adults. Hum Vaccin Immunother. 2013 Sep;9(9):1962-70.
Non Patent Literature 3: Misumi S, Masuyama M, Takamune N, et al. Targeted Delivery of Immunogen to Primate M Cells with Tetragalloyl Lysine Dendrimer Journal of Immunology, 2009;182(10):6061-6070

### Summary of Invention

### Technical Problem

The present invention relates to a provision of an influenza vaccine having high efficacy in intranasal administration.

### Solution to Problem

As a result of intensive studies, the present inventors have found that in a case where an influenza antigen to which the M cell-targeting molecule TGDK is linked via a chemical bond is administered through the nasal mucosa, the immune response in the blood and nasal cavity is enhanced, and the virus antigen is useful as an influenza vaccine for intranasal administration.

In other words, the present invention relates to 1) to 5) below.
1) An influenza vaccine composition to be intranasally administered, containing an influenza antigen to which a TGDK is linked via a chemical bond.
2) The vaccine composition according to 1), in which the influenza antigen is an antigen of one or more viruses selected from an influenza A virus strain and an influenza B virus strain.
3) The vaccine composition according to 1) or 2), in which the influenza antigen is a split antigen or a whole inactivated virus antigen.
4) The vaccine composition according to any one of 1) to 3) that is formulated as a liquid agent or dry powder.
5) The vaccine composition according to any one of 1) to 4), in which a content of the influenza antigen in the composition is 1 to 21 µg HA/strain.
6) An immunization method including intranasally administering an influenza vaccine composition containing an influenza antigen to which a TGDK is linked via a chemical bond to a subject in need thereof.
7) An influenza prevention method including intranasally administering an influenza vaccine composition containing an influenza antigen to which a TGDK is linked via a chemical bond to a subject in need thereof.

### Advantageous Effects of Invention

According to the present invention, it is possible to provide an influenza vaccine that has a high efficacy and is capable of inducing the mucosal immunity, and it is a great contribution to pharmaceutical industry by creating an influenza preventive drug with a high value added.

### Brief Description of Drawings

Fig. 1 shows an evaluation of transcytosis in an in vitro M-like cell model.
Fig. 2 shows an IgG antibody titer in serum against an A/Switzerland/9715293/2013 strain (an A/H3N2 subtype).
Fig. 3 shows an IgA antibody titer in nasal wash against an A/Switzerland/9715293/2013 strain (an A/H3N2 subtype) on Day 56.
Fig. 4 shows evaluation of transcytosis efficiency from M cells of a mucous membrane.
Fig. 5 shows an IgG antibody titer in serum against an A/California/07/2009 strain (an A/H1N1 subtype) on Day 35.
Fig. 6 shows an IgG antibody titer in serum against an A/California/07/2009 strain (an A/H1N1 subtype) on Day 56.
Fig. 7 shows an IgA antibody titer in nasal wash against an A/California/07/2009 strain (an A/H1N1 subtype) on Day 56.

### Description of Embodiments

Hereinafter, preferred embodiments of the present invention will be described in detail. However, the present invention is not limited to the embodiments below.

In the present invention, the term "influenza vaccine" means a vaccine containing at least an antigen of either influenza A virus or influenza B virus. In other words, the influenza vaccine may be a monovalent vaccine containing either one of influenza A virus or influenza B virus, or may be a multivalent vaccine containing both of them. Examples of the influenza vaccine of the present invention include a trivalent or higher valent vaccine containing at least three or more types of influenza virus strains, for example, two types of influenza A virus strains and one or two types of -influenza B virus strains, and preferably include a tetravalent vaccine. More preferably, a tetravalent vaccine containing two types of A strains (an A/H1N1 subtype strain and an A/H3N2 subtype strain) and two types of B strains (a B/Victoria lineage strain and a B/Yamagata lineage strain) can be exemplified.

In the present invention, the term "influenza virus" refers to influenza A virus, influenza B virus, or both of them. Furthermore, the influenza virus also includes all currently known subtypes and subtypes to be isolated and identified in future.

The influenza virus strain used for preparing the vaccine of the present invention may be a strain isolated from an infected animal or patient, or may be a recombinant virus established in cultured cells by genetic engineering.

The virus antigen in the influenza vaccine composition of the present invention is an influenza antigen to which a TGDK is linked via a chemical bond.

Examples of the influenza antigen include a whole inactivated virus (a whole inactivated virus antigen) having infectivity eliminated while a form of virus particles is maintained, a split virus (a split antigen) obtained by disrupting the virus particles with a surfactant or an organic solvent such as diethyl ether, and subunit antigens.

The subunit antigens refer to a component derived from the influenza virus, examples thereof include hemagglutinin (HA), neuraminidase (NA), matrixes (M1 and M2), non-structure (NS), polymerase (basic polymerase (PB1 and PB2) and acidic polymerase (PA)), and nucleoprotein (NP), and among the subunit antigens, the HA and the NA are preferable. The subunit antigens may be prepared from a natural virus or may be produced by synthetic or recombinant techniques.

The influenza virus particles can be prepared by using an embryonated chicken egg method or a cell culture method.

The "embryonated chicken egg method" refers to a method including inoculating an embryonated chicken egg with a virus strain and culturing the resultant, and thereafter clarifying, concentrating, purifying, and inactivating the virus suspension to thereby obtain a virus solution containing virus particles. Furthermore, the "cell culture method" refers to a method including inoculating cultured cells with a virus strain and culturing the resultant, and thereafter obtaining a virus solution containing virus particles from the culture supernatant in a similar manner as in the embryonated chicken egg method described above. The cultured cells are not particularly limited as long as the influenza virus exhibits proliferation property, and examples thereof include MDCK (Madin-Darby Canine Kidney), Vero, Caco-2, PER.C6, EB66, and recombinant cells thereof that have been recombined so as to highly express receptors used for entry by the virus.

Here, the cultured cells are inoculated with the influenza virus strain and cultured at 30 to 37°C for about 1 to 7 days, preferably at 33 to 35°C for about 2 days. After completion of the culture, the virus suspension (an infected allantoic fluid or an infected cell culture supernatant) is collected and centrifuged or filtered for clarification. Next, for concentration, a barium salt adsorption elution reaction or ultrafiltration is performed. The purification of the virus can be performed by using a means including ultracentrifugation such as sucrose density gradient centrifugation, or liquid chromatography.

The virus solution purified is inactivated. Examples of a virus inactivation method include formalin treatment, ultraviolet irradiation, and treatment with beta-propiolactone, binary ethyleneimine or the like.

In the present invention, the term "TGDK" refers to an abbreviation for tetragalloyl-D-lysine dendrimer, and refers to N²,N⁶-bis[N²,N⁶-bis(3,4,5-trihydroxybenzoyl)-lysyl]-N-(2-aminoethyl)-lysine amide. TGDK is known as a target molecule for the M cells that are antigen uptake cells present in the mucous membrane.

TGDK can be produced, for example, by a solid phase or liquid phase synthesis by using a gallic acid and D-lysine (Patent Literatures 1 and 2 as well as Non Patent Literature 3).

The chemical bond between TGDK and the influenza antigen may be either a bond formed via a crosslinker or a direct covalent bond between the influenza antigen and TGDK.

In a case where a crosslinker is used, TGDK can be linked via a terminal primary amine, and the influenza antigen can be linked via a protein, a sugar chain, and a lipid. In a case of linking via the protein, a primary amine, a carboxyl group, and a sulfhydryl group are targeted, and a crosslinker having, at a terminal, an NHS ester, an imide ester, a pentafluorophenyl ester, and hydroxymethyl phosphine can be used in a case of linking via the primary amine, a crosslinker having, at a terminal, carbodiimide in a case of linking via the carboxyl group, and a crosslinker having, at a terminal, maleimide, haloacetic acid, pyridyl disulfide, thiosulfone, and vinyl sulfone in a case of linking via the sulfhydryl group. In a case of linking via the sugar chain, a glycol of the sugar chain is oxidized with, for example, sodium periodate, a carbonyl group thus generated becomes a target, and a crosslinker having hydrazide and alkoxyamine at a terminal can be used. Furthermore, in a case of linking via the lipid, a crosslinker having a linear or branched carbon chain can be used, and examples thereof include commercially available DSPE-PEG (5000) Maleimide (Merck) .

The crosslinker to be used may be a homobifunctional crosslinker having the same terminal reactive group or a heterobifunctional crosslinker having different terminal reactive groups. Furthermore, as in a click chemistry method, cyclooctyne may be modified on one of the influenza antigen and TGDK, an azide group may be modified on the other, and the influenza antigen and TGDK may be linked to each other by mixing to form a triazole ring with the cyclooctyne and the azide group, for example, DBCO-PEG-NHS (Biochempeg) and Azido-PEG4-NHS Ester (Tokyo Chemical Industry Co., Ltd.) can be used for linking the influenza antigen and TGDK.

In a case of direct linking, the primary amine of TDGK and the carboxyl group present in the protein of the influenza antigen can be condensed and linked via amide formation. As a condensation reaction, a Schotten-Baumann reaction may be used in which the carboxyl group is made to be an acid chloride and then reacted with the primary amine in the presence of a base to form an amide, or a reaction using a condensing agent including a carbodiimide-based condensing agent such as DCC; BOP, HBTU, HATU, COMU, or a triazole-based condensing agent may be used.

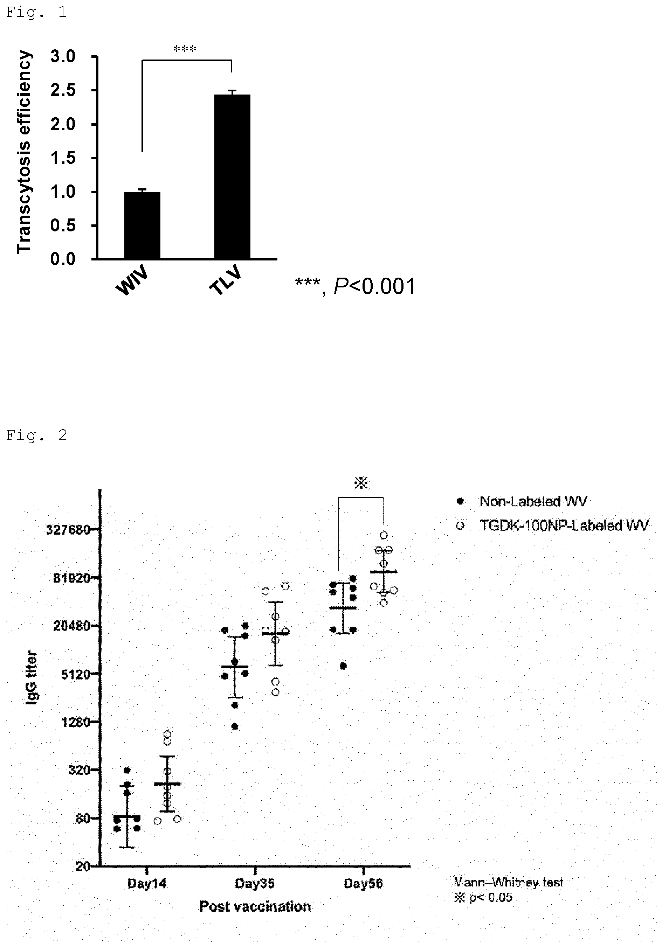

As shown in examples to be described later, a significant improvement in the transcytosis efficiency has been confirmed by linking of TGDK to the influenza antigen in the in vitro M-like cell model (Fig. 1). Furthermore, in a mouse immunogenicity test, a significant increase in antibody titer has been confirmed in both the IgG in blood and the IgA in the nasal wash for the influenza antigen to which TGDK is linked as compared to an influenza antigen without TGDK.

Therefore, the influenza antigen to which TGDK is linked via the chemical bond can be the influenza vaccine for intranasal administration, and can be used to perform efficient immunity induction by the intranasal administration.

Furthermore, intranasal administration of the influenza antigen to which TGDK is linked via the chemical bond to a subject (a human and a mammal other than the human) is useful as an immunization method for inducing an antibody to the subject and as the influenza prevention method.

The influenza vaccine composition of the present invention contains, in addition to the influenza antigen, a pharmaceutically acceptable carrier as appropriate, and can be formulated into a predetermined form.

Here, examples of the carrier include a carrier usually used for producing the vaccine, and specific examples thereof include a buffer, an emulsifier, a preservative (for example, thimerosal), an isotonizing agent, a pH adjusting agent, a thickening agent, an inactivating agent (for example, formalin), an adjuvant, or an immunostimulant. The adjuvant refers to a substance that enhances the immune response to an antigen by administering the adjuvant together with the antigen, but since the vaccine antigen is the influenza antigen to which TGDK is linked via a chemical bond as described above and has high antibody inducibility, the adjuvant is not necessarily added and the vaccine composition of the present invention may be an adjuvant-free composition.

Such influenza vaccine composition can be formulated as a composition (a pharmaceutical composition) for intranasal administration (administration through a nasal mucosa) in a solid, semi-solid or liquid form.

A dosage form of the composition may be any form that can be dissolved or disintegrated on the nasal mucosa, and can be dry powder or a liquid agent (in a form of a solution, a suspension, an amorphous gel, or the like). Furthermore, the administration to the nasal mucosa is not limited, and various methods such as spraying, coating, and dropping can be adopted.

The influenza vaccine composition of the invention is preferably formulated as the liquid agent for administration as an aerosol or a nose drop, or formulated as a lyophilized powder to be rapidly deposited in a nasal passage.

Examples of the liquid agent in a form of a solution include a solution obtained by dissolving the influenza vaccine composition in purified water or a buffer solution, and examples of the liquid agent in a form of a suspension include a suspension obtained by suspending the influenza vaccine composition in, for example, purified water or a buffer solution together with methyl cellulose, hydroxymethyl cellulose, polyvinyl pyrrolidone, gelatin, casein, or the like. Furthermore, in addition to a mucoadhesive substance, a filler and an active substance (for example, mannitol, sucrose, trehalose, and xylitol) for exhibiting appropriate powder flow and size characteristics can be appropriately blended in a dry powder pharmaceutical composition.

A content of the influenza antigen in the vaccine composition is 1 µg or more per virus strain, that is, 1 µg HA/strain or more, preferably 1 to 21 µg HA/strain, and more preferably 15 µg HA/strain, as an amount of hemagglutinin. Note that, the amount of hemagglutinin is a value obtained by measurement by a test method defined in accordance with WHO or national standards, such as a single radial immunodiffusion test method. Furthermore, a content of the antigen contained in the vaccine may be appropriately varied according to the type of the virus or the subject to be administrated.

Examples of the subject to be administrated with the influenza vaccine of the present invention include humans and mammals other than humans, and the humans are preferable. Examples of the mammals other than humans include mice, rats, guinea pigs, rabbits, pigs, cows, horses, goats, sheep, dogs, cats, rhesus monkeys, cynomolgus monkeys, orangutans, and chimpanzees.

The number of times for administering the influenza vaccine of the present invention is at least once, but is preferably at least twice from a viewpoint of efficacy. Further administration is sometimes referred to as a booster, and thus a more effective infection protection effect can be achieved. An interval of the booster is recommended to be at least 1 week, and an interval of 1 to 4 weeks is preferable.

### Examples

Hereinafter, the present invention will be specifically described with reference to examples, but the present invention is not limited thereto at all.

### Example 1 Evaluation of transcytosis in in vitro M-like cell model

Preparation of whole inactivated antigen used in the present example is as described below. A virus of the A/California/07/2009 strain (A/H1N1 pdm9) was inoculated into a chorioallantoic cavity of a 12-day-old embryonated chicken egg, and cultured for 3 days, and the chorioallantoic fluid was sampled. The chorioallantoic fluid sampled was clarified by filter filtration, then adsorbed to barium sulfate, and eluted with a 12% sodium citrate solution to collect the influenza virus. The virus collected was further purified by replacing the solution with 6.7 mM phosphate buffered saline (pH 7.2) by ultrafiltration, and collecting a fraction containing the influenza virus by sucrose density gradient centrifugation after buffer replacement. Beta-propiolactone as an inactivating agent was added to the influenza virus purified to have a final concentration of 0.05%, and infectivity of the influenza virus was inactivated by an inactivation reaction at 4°C for 24 hours. After the inactivation reaction, the buffer was replaced with 6.7 mM phosphate buffered saline (pH 7.2) containing sucrose of 1 w/w% by the ultrafiltration (MWCO: 100,000) to prepare the whole inactivated vaccine.

Azido-PEG4-NHS ester (Tokyo Chemical Industry Co., Ltd.) dissolved in 0.25 mL of phosphate buffered saline was added to 1 mL of the whole inactivated vaccine, adjusted to have a pH of 8.0 with HEPES, and then stirred for 3 hours. After being stirred, 1 mL of 1 M Tris-HCl buffer (pH 7.5) was added to stop the reaction. After the reaction was stopped, a centrifugation was performed at 82,000 × g for 45 minutes, and a precipitate obtained was suspended in 0.5 mL of phosphate buffered saline. A similar centrifugation was repeated again for washing, and a suspension obtained was regarded as an azidated whole inactivated vaccine.

TGDK prepared by the method described in Patent Literature 2 was dissolved in N,N-dimethylformamide (DMF), and mixed with a DBCO-PEG-NHS (5K) ester (Biochempeg) and then, a pH was adjusted to 8.0. After the pH was adjusted, stirring was performed for 3 hours, then ether was added, followed by centrifugation at 3,000 × g. A precipitate obtained was freeze-dried, and regarded as TGDK-DBCO5K.

The TGDK-DBCO5K dissolved in phosphate buffered saline was added to the azidated whole inactivated vaccine and stirred overnight. After being stirred, the centrifugation was performed at 82,000 × g for 45 minutes, the precipitate obtained was suspended by adding 1 mL of phosphate buffered saline, and a suspension thus obtained was regarded as a TGDK-linked whole inactivated vaccine (a TGDK-Labeled virus: TLV).

As described in a literature (Kai H, Motomura Y, Saito S, Yamamoto K, Tatefuchi T, Takamune N, Misumi S. Royal jelly enhances antigen-specific IgA response. Food Sci Nutr. 2013 Mar 6; 1(3): 222-227), 3 × 10⁵ Caco-2 cells were seeded on a transwell (Corning, a pore size of 3 µm, 24-well) membrane, allowed to stand overnight, and then the transwell membrane was immersed in a 24-well plate added with Eagles MEM containing 20% fetal bovine serum and 0.1 mM non-essential amino acid (20% FBS, 0.1 mM NEAA EMEM). The culture was performed for 21 days while the transwell was transferred to the 24-well plate newly added with the 20% FBS, 0.1 mM NEAA EMEM approximately every 3 days to form a Caco-2 monolayer. After being cultured for 21 days, 1 × 10⁶ Raji B cells were added to an upper chamber of the transwell, and further cultured for 3 days to promote differentiation into M-like cells.

As described above, the cells that were promoted to differentiate into M-like cells in vitro were prepared on the transwell membrane, the whole inactivated vaccine (whole inactivated virus: WIV) or TGDK-liked whole inactivated vaccine (TGDK-Labeled virus: TLV) was added to a lower chamber of the transwell, and an amount of an influenza virus genome contained in an upper chamber solution was measured by qPCR to evaluate an amount of the whole inactivated vaccine transferred to the upper chamber after 1 to 3 hours from the addition. Note that, from measurement results of the amount of the viral genome, a ratio of the TLV to the WIV was determined and regarded as the transcytosis efficiency.

The measurement results of the amount of the viral genome are illustrated in Fig. 1, but for the TLV, the transcytosis efficiency was 2.5 times higher than the transcytosis efficiency of the WIV, and a significant improvement in the transcytosis efficiency was confirmed. Therefore, it was considered that TGDK exerts a function of promoting an uptake via M cells and promotes an uptake of the influenza antigen through a mucous membrane even when linked to the influenza antigen.

### Example 2 Mouse immunogenicity test

The whole inactivated vaccine was prepared by the method described in Example 1, and the A/Switzerland/9715293/2013 strain was used as the virus. Furthermore, TGDK was also prepared by the method in Patent Literature 2 in a similar manner as in Example 1.

790 µg of TGDK was dissolved in dehydrated dimethyl sulfoxide, and adjusted to have a volume of 0.5 mL. Furthermore, SUNBRIGHT PTE-100NP (NOF CORPORATION) was dissolved in the dehydrated dimethyl sulfoxide to be 1 mM, and 200 µL of a TGDK solution was added to 100 µL of the SUNBRIGHT PTE-100NP solution and gently stirred for 2 hours. 300 µL of the solution after being stirred was added to 2.7 mL of the whole inactivated vaccine, and stirred at a room temperature for 16 hours. After being stirred, adjustment was performed to have a pH of 8.0, and 50 µL of 1 M Tris-HCl buffer (pH 7.5) was further added to stop the reaction. After the reaction was stopped, 1.5 mL of phosphate buffered saline containing sucrose of 10% was added, and 6 mL of phosphate buffered saline was further added. The solution was subjected to buffer exchange into phosphate buffered saline containing sucrose of 1% by the ultrafiltration (MACROSEP OMEGA: POLL), and regarded as a TGDK-100NP-Labeled WV.

The TGDK-linked influenza vaccine (the TGDK-100NP-Labeled WV) prepared and the whole inactivated influenza vaccine (Non-Labeled WV) as a control were administered to a BALB/c mouse (female, 5 weeks old) intranasally in an amount of 0.01 mL (containing 1 µg HA as an antigen amount) per mouse 3 times at intervals of 3 weeks, blood collection and serum preparation were performed 2 weeks after each administration (on Day 14, 35, and 56), and on Day 56, the nasal wash was collected after the blood collection. The serum collected was subjected to measurement of an antigen-specific IgG antibody titer by ELISA, and the nasal wash was used to measure the antigen-specific IgA antibody titer by the ELISA.

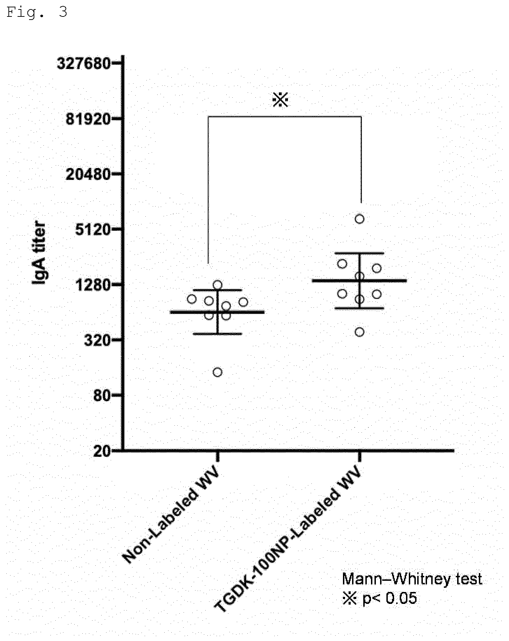

The results of the measurement of the antibody titer were as illustrated in Figs. 2 and 3, the antigen-specific IgG antibody titer in serum showed high antibody induction in the vaccine to which the TGDK was linked at any time point, and it was found that the antibody induction was significantly improved by the linking of TGDK particularly on Day 56. Furthermore, also with respect to the antigen-specific IgA antibody titer at the same time point, the vaccine to which the TGDK was linked showed a significantly higher antibody titer than the control.

Therefore, it was confirmed that the influenza vaccine for intranasal administration in which TGDK is linked to the influenza antigen improved the uptake through the mucous membrane as compared with the vaccine without TGDK, and thus improved the antibody induction in the blood and the nasal cavity. It can be said that such influenza vaccine for intranasal administration is a vaccine that is high in safety since the inactivated antigen is used, and also high in immunogenicity due to the functions of TGDK.

### Comparative Example 1

Synthesis of TGDK and production of M-like cells were performed by the method described in Example 1, and the transcytosis was evaluated under conditions in which the TGDK was not linked.

As described above, the cells that were promoted the differentiation into M-like cells in vitro were prepared on the transwell membrane, and 22.9 µg of the whole inactivated vaccine (: Virus) as an amount of an HA antigen, the same amount of the whole inactivated vaccine and 1.1 µM of free TGDK (: Virus + TGDK (low)), as well as the same amount of the whole inactivated vaccine and 11.1 µM of free TGDK (: Virus + TGDK (high)) were added to the lower chamber, and the amount of the influenza virus genome contained in a solution in the upper chamber was measured by qPCR in order to evaluate the amount of the whole inactivated vaccine transferred to the upper chamber after 1 hour. Note that, in the experiment, a similar experiment was also performed on a Caco-2 monolayer (without addition of the Raji B cells) that was not differentiated into M-like cells as a control in order to evaluate the transcytosis efficiency from the M cells of the mucous membrane.

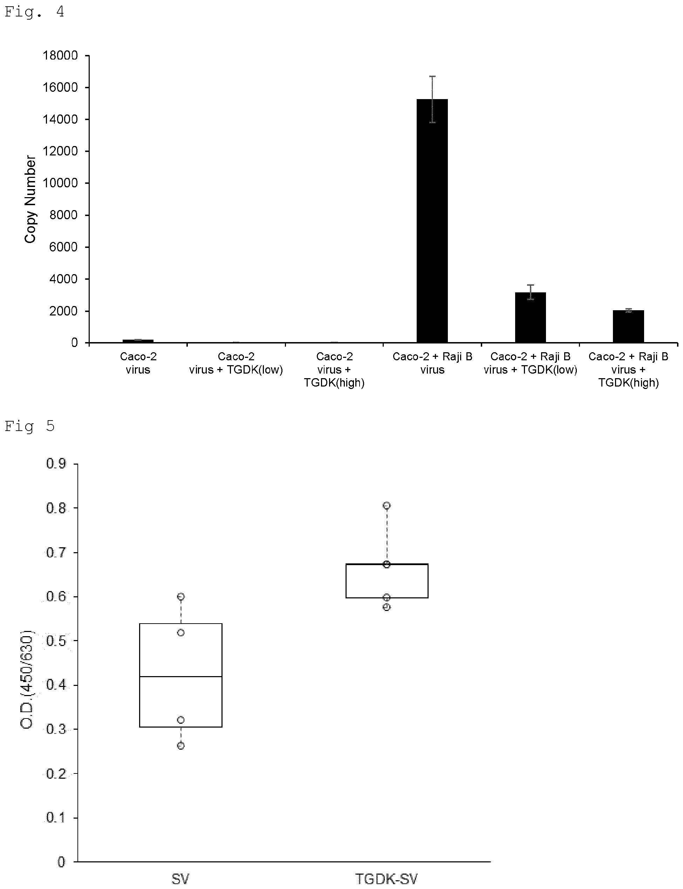

As illustrated in Fig. 4, since the transcytosis does not work on the Caco-2 monolayer that has not been differentiated into the M-like cells, a transfer of the vaccine antigen to the upper chamber was not confirmed under any conditions. Furthermore, in M-like cells (Caco-2 + Raji B), the transfer of the vaccine antigen to the upper chamber was confirmed in the whole inactivated vaccine alone, whereas the transfer was inhibited by adding TGDK, and as a result, an inhibitory effect correlated with the concentration of TGDK.

Therefore, as shown in Example 1, it was shown that the linking of TGDK to the whole inactivated vaccine promotes the transfer to the upper chamber in M-like cells, that is, promotes the uptake by the transcytosis from the mucous membrane, but when TGDK and the vaccine antigen coexist without being linked, the transcytosis of the vaccine antigen is inhibited. It is considered that since TGDK is preferentially linked to the surface of M cells and taken up, the uptake of the virus particles is reduced in a case where TGDK as the M cell-targeting molecule and the vaccine antigen coexist. In other words, in a case where it is desired to promote the uptake of the vaccine antigen through the mucous membrane, it is considered that TGDK needs to be linked to the vaccine antigen.

### Reference Example 1

An amount of TGDK linked of the TGDK-linked influenza vaccine (TGDK-100NP-Labeled WV) prepared in Example 2 was evaluated by an iron tartrate method. Details of the measurement by the iron tartrate method are as described below.

In 0.1 M 2-morpholinoethanesulfonic acid (: MES), 12.5 mg/mL of sodium dodecyl sulfate, 2.5 mg/mL of sodium potassium tartrate tetrahydrate, and 0.5 mg/mL of iron (II) sulfate heptahydrate were dissolved as a final concentration, and regarded as an iron tartrate test solution. As a standard substance for a calibration curve, ethyl gallate was prepared to be 15.6 to 500 µM in 0.1 M MES, the standard substance for a calibration curve, samples and 180 µL of 0.1 M MES as a blank were taken, 20 µL of the iron tartrate test solution was added thereto, then 12 µL of 2 M trishydroxymethylaminomethane was added, and absorbances of this reaction solution at wavelengths of 520 nm and 660 nm were measured. The calibration curve was generated from the difference in the absorbances at respective wavelengths, and the difference in the absorbances of respective samples was substituted into the calibration curve to calculate a quantitative value of a galloyl group. Note that, the samples were the TGDK-linked influenza vaccine (TGDK-100NP-Labeled WV), and a concentration of the antigen was 1307 µg HA/mL.

Measurement results showed that the galloyl group was contained with the concentration of 139 µM (139 nmol/mL), which was 0.11 nmol/ug HA in terms of the amount of the HA antigen as a main antigen. However, since the TGDK has 4 galloyl groups in a molecule, the concentration of the galloyl group in terms of the TGDK is 0.026 nmol/µg HA, that is, 0.03 µg/µg HA (a molecular weight of TGDK: 1053). In Example 2, since 1 µg HA was administered per an animal, it can be seen that for the TGDK-100NP-Labeled WV, 0.03 µg of TGDK linked to the vaccine was administered per an animal.

### Example 3

In the present example, a stock solution of the A/H1N1 subtype (the A/California/07/2009 strain) of an influenza HA vaccine "SEIKEN" was regarded as the split antigen.

SUNBRIGHT (8)-HGEO-200NP of 2 mg was dissolved in N,N-dimethylformamide, mixed with a TGDK solution (5 nmol/mL) to which N-methylmorpholine was added, and reacted for 3 hours. After the reaction, color change of the reaction liquid to yellow due to para-nitrophenol release was confirmed, and the diethyl ether was added to the reaction liquid to obtain a TGDK-linked SUNBRIGHT (8)-HGEO-200NP precipitate. The precipitate was again washed with diethyl ether and the precipitate obtained was regarded as TGDK-linked SUNBRIGHT (8)-HGEO-200NP. Furthermore, 1 mL of 6.7 mM Na2HPO4 (pH 9.6) was added to a split antigen solution (with a protein content: 829 µg) according to the above, and adjusted to have a pH of 7.8 to 7.9. The TGDK-linked SUNBRIGHT (8)-HGEO-200NP was added to the split antigen solution with the pH adjusted, and reacted at the room temperature for 3 hours. After the reaction, diethyl ether was added to remove para-nitrophenol as a reaction byproduct, and a lower layer containing a TGDK-linked split antigen (TGDK-SV) was collected. Diethyl ether was added again to the lower layer collected to remove impurities, and the lower layer was freeze-dried to obtain a purified TGDK-linked split antigen.

The TGDK-SV prepared of 0.01 mL (containing the antigen in an amount of 1.5 µg HA) was intranasally administered to BALB/c mouse (female, 5 weeks old, N = 5) 3 times at intervals of 3 weeks, and the blood collection and the serum preparation were performed after 2nd and 3rd administrations (on Day 14, 35, and 56). Furthermore, on Day 56, the nasal wash was collected after the blood collection. The serum collected was subjected to measurement of an antigen-specific IgG antibody titer by ELISA, and the nasal wash was used to measure the antigen-specific IgA antibody titer by the ELISA. Note that, as a control, the same amount of the split antigen (SV) was administered.

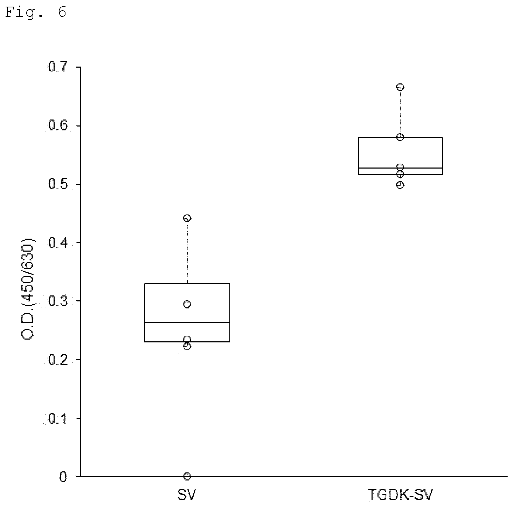

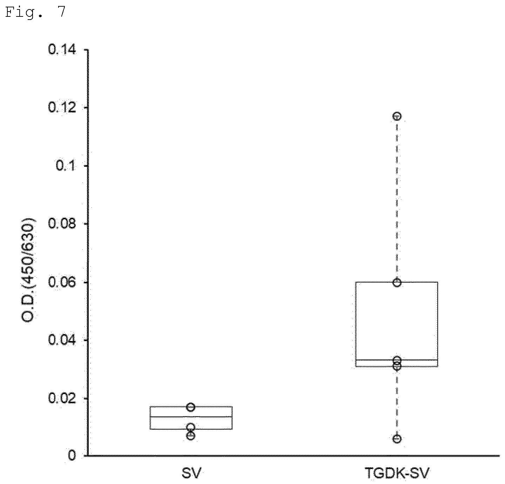

Results of an influenza-specific IgG in the serum on Day 35 are as illustrated in Fig. 5, and the antibody induction in the serum in the second administration showed a tendency to be higher in the TGDK-SV, and similarly, the IgG in the serum was also higher in the TGDK-SV than in the SV alone on Day 56 after a third administration (Fig. 6). Furthermore, as illustrated in Fig. 7, the IgA in the nasal wash on Day 56 also showed a tendency to have a higher antibody titer in the TGDK-SV than in the SV, and it was confirmed that the immunogenicity of the SV in the intranasal administration was improved by the linking of the TGDK.

## Claims

1. An influenza vaccine composition to be intranasally administered, comprising an influenza antigen to which TGDK is linked via a chemical bond.

2. The vaccine composition according to claim 1, wherein the influenza antigen is an antigen of one or more selected from an influenza A virus strain and an influenza B virus strain.

3. The vaccine composition according to claim 1 or 2, wherein the influenza antigen is a split antigen or a whole inactivated virus antigen.

4. The vaccine composition according to any one of claims 1 to 3, wherein the vaccine composition is formulated as a liquid agent or dry powder.

5. The vaccine composition according to any one of claims 1 to 4, wherein a content of the influenza antigen in the composition is 1 to 21 µg HA/strain.

6. An immunization method comprising intranasally administering an influenza vaccine composition containing an influenza antigen to which TGDK is linked via a chemical bond to a subject in need thereof.

7. An influenza prevention method comprising intranasally administering an influenza vaccine composition containing an influenza antigen to which TGDK is linked via a chemical bond to a subject in need thereof.
